# EUROPÄISCHE PATENTSCHRIFT

(11) **EP 3 796 778 B1**
(45) Veröffentlichungstag und Bekanntmachung des Hinweises auf die Patenterteilung: **01.10.2025**
(21) Anmeldenummer: 19722503.0
(22) Anmeldetag: 03.04.2019
(51) Int. Cl.: A61M 1/02, A61M 5/44, F28D 21/00, H05B 1/00

(54) **TEMPERIERVORRICHTUNG UND TEMPERIERVERFAHREN**
TEMPERATURE-CONTROL DEVICE AND TEMPERATURE-CONTROL METHOD
DISPOSITIF DE THERMORÉGULATION ET PROCÉDÉ DE THERMORÉGULATION

(30) Priorität: 22.05.2018 DE 102018112194
(43) Veröffentlichungstag der Anmeldung: 31.03.2021
(73) Patentinhaber: Barkey GmbH & Co. KG, 33818 Leopoldshöhe (DE)
(72) Erfinder: BALLUFF, Michael, 33619 Bielefeld (DE); REMPEL, Harry, 33818 Leopoldshöhe (DE); PETRACH, Christian, 32602 Vlotho (DE)
(74) Vertreter: Ostermann, Thomas
(86) Internationale Anmeldenummer: PCT/DE2019/100306
(87) Internationale Veröffentlichungsnummer: WO 2019/223828

(56) Entgegenhaltungen:
- WO-A1-2016/023034
- WO-A1-2017/153761
- JP-B2- 2 936 108
- US-A1- 2015 122 793

## Beschreibung

Die Erfindung betrifft eine Vorrichtung zum Temperieren und Auftauen von einem Temperiergut mit einem Gehäuse, in dem das Temperiergut, ein Heizmodul zur Einbringung von Wärme an mindestens einer Seite des Temperiergutes, ein Stellorgan, mittels dessen das Temperiergut in Bewegung bringbar ist. Aus der US 6 748 164 B1 ist eine Vorrichtung zum Auftauen eines Temperiergutes bekannt, bei dem das Temperiergut von einem Heizorgan umgeben ist. In dem Heizorgan wird warme Flüssigkeit umgewälzt. Durch den direkten thermischen Kontakt zwischen dem Heizorgan und dem Temperiergut erfolgt ein Wärmeübergang von dem Heizorgan in das Temperiergut. Zur Beschleunigung des Auftauvorganges ist als Stellorgan ein periodisch hoch und runter bewegbarer Gehäuseboden, auf dem das Heizorgan angeordnet ist. Damit wird das Heizorgan in eine Schwenkbewegung versetzt, wobei eine Schwenkachse entlang eines Randes des Heizorgans verläuft. Das Temperiergut wird damit mittelbar verschwenkt, wobei eine Randseite schwenkachsennah angeordnet ist.

Aus der US 8 012 416 B2 ist eine Vorrichtung zum Auftauen eines Temperiergutes bekannt, wobei das Temperiergut an gegenüberliegenden Seiten jeweils mit einem Heizorgan in Kontakt steht. Das untere Heizorgan ist auf mehreren Fußplatten angeordnet, denen jeweils aufblasbare Kissen zugeordnet sind. Die Kissen werden antizyklisch aufgeblasen, so dass bezogen auf eine Mittelebene die eine Hälfte des Heizorgans einerseits und dann die andere Hälfte des Heizorgans andererseits angehoben wird. Damit lässt sich das Temperiergut bezogen auf die Mittelebene zuerst auf einer ersten Hälfte und dann auf einer zweiten Hälfte zusammendrücken. Nachteilig an der bekannten Vorrichtung ist, dass der Aufwand für das temporäre und partielle Zusammendrücken des Temperiergutes relativ aufwendig ist. Die Frequenz bzw. Beschleunigung ist relativ begrenzt.

Aus der EP 0 318 924 B1 ist eine Vorrichtung zum Temperieren und Auftauen von einem Temperiergut bekannt, bei dem das Temperiergut zwischen zwei Heizorganen eines Heizungsmoduls angeordnet ist. Die Heizorgane sind als Kunststoffbeutel ausgebildet, in denen eine warme Flüssigkeit mittels einer Pumpe umgewälzt wird. Randseitig der Heizorgane ist eine Kurbel angeordnet, die mittels eines Motors in Drehung versetzt wird und abwechselnd den oberen Heizbeutel und den unteren Heizbeutel randseitig zusammendrückt, so dass die innerhalb der Heizbeutel angeordnete warme Flüssigkeit von der Kurbel wegbewegt wird. Hierdurch entstehen Bewegungsschwingungen innerhalb des Heizbeutels, die an das Temperiergut übertragen werden können.

Aus der WO 2017/153761 A1 ist eine Vorrichtung zum Temperieren und Auftauen von einem Temperiergut bekannt, das ein Heizmodul zur Einbringung von Wärme in das Temperiergut vorsieht. Das Heizmodul umfasst eine Heizplatte sowie eine Mehrzahl von linear und senkrecht zu der Erstreckungsebene des Temperiergutes bewegbare Heizstößel. Die Heizstößel sind unabhängig voneinander beweglich, so dass unterschiedliche Bereiche des Temperiergutes mit einem unterschiedlichen Bewegungsimpuls beaufschlagt werden können. Über den Kontakt der Heizstößel an das Temperiergut wird die Wärme auf das Temperiergut übertragen.

Aus der US 2015/0122793 A1 ist eine Vorrichtung zum Temperieren und Auftauen von einem Temperiergut bekannt, die ein Heizmodul mit einem ersten Heizelement und einem zweiten Heizelement vorsieht. Das erst Heizelement ist hin und her bewegbar. Das zweite Heizelement ist über eine Achse schwenkbar gelagert, so dass es eine Druckkraft auf das Temperiergut an gegenüberliegenden Seiten des Temperiergutes ausüben kann.

Aufgabe der vorliegenden Erfindung ist es, eine Vorrichtung zum Temperieren und Auftauen von einem Temperiergut derart weiterzubilden, dass auf einfache Weise die Effektivität des Temperierens weiter verbessert wird.

Zur Lösung dieser Aufgabe weist die Erfindung die Merkmale des Patentanspruchs 1 auf.

Die erfindungsgemäße Vorrichtung ermöglicht durch die direkte Anlage eines mechanischen Stellelementes an dem Temperiergut eine gezielte und unmittelbare Bewegungssteuerung des Temperiergutes, so dass eine Wärmeeinleitung von dem Heizorgan in das Temperiergut beschleunigt werden kann. Vorteilhaft kann die von dem Stellelement vorgegebene Bewegung direkt und unmittelbar auf das Temperiergut übertragen werden. Unerwünschte Störungen bei der Bewegungsvermittlung von dem Stellelement zu dem Temperiergut durch andere Bauteile können vermieden werden. Die Erfindung ermöglicht insbesondere einen relativ schnellen Austausch eines in einem Außenbereich angeordneten üblicherweise erwärmten Teils des Temperiergutes mit einem in einem Kernbereich desselben angeordneten üblicherweise kühlen Teils des Temperiergutes. Es ist somit eine relativ schnelle Durchmischung des Temperiergutes gewährleistet. Das Temperiergut kann beispielsweise als ein Fluid oder Gel ausgebildet sein, das in einer Umverpackung angeordnet ist. Die Erfindung ermöglicht somit eine relativ schnelle Durchmischung des Fluids bzw. Gels. Die Erwärmung des Temperiergutes kann durch Konduktion oder durch Strahlung (Infrarotstrahlung, Mikrowelle) oder durch Luftströmung erfolgen. Nach der Erfindung ist das mechanische Stellelement mechanisch direkt gekoppelt mit dem Temperiergut. Es kann somit eine unmittelbare Einleitung vorzugsweise eines stoßartigen Bewegungsimpulses partiell an dem Temperiergut erfolgen.

Nach der Erfindung ist das mechanische Stellelement derart ansteuerbar, dass eine Schwenkbewegung erfolgt. Das Temperiergut wird hierbei partiell in unterschiedlichen Wegstrecken aus ihrer Erstreckungsebene ausgelenkt. Das Temperiergut wird verformt bzw. "geknetet", was zu einer inneren Strömung bzw. Bewegung des gefrorenen Kerns sowie des bereits aufgetauten Anteils des Temperiergutes führt. Vorzugsweise verläuft die Schwenkachse im Bereich einer Mittelebene bzw. Quermittelebene des Temperiergutes, so dass das Temperiergut nach Art eines Paddels eines Ruderbootes um seine Mittelachse hin und her verschwenkt wird. Vorteilhaft kann hierdurch der gefrorene Teil des Temperiergutes sowie des bereits aufgetauten Anteils ständig in einer Hin- und Herbewegung in dem bereits geschmolzenen Teil des Temperiergutes bewegt werden. Es entstehen hierdurch inhomogene Strömungen innerhalb des Temperiergutes, wobei kalte Flüssigkeit an die Oberfläche des Temperiergutes strömt und dadurch ein höherer Temperaturgradient vom Heizelement zum Temperiergut entsteht. Es hat sich gezeigt, dass sich hierdurch die Auftauzeit um 30 % reduzieren lässt.

Nach einer bevorzugten Ausführungsform der Erfindung ist das mechanische Stellelement flächig ausgebildet. Es kann der Formgebung des Temperiergutes angepasst ausgebildet sein, so dass ein linienförmiger und/oder flächiger Kontakt zwischen dem Stellelement und dem Temperiergut besteht. Vorzugsweise ist das Stellelement eben ausgebildet, so dass es platzsparend zwischen dem Temperiergut und dem Heizorgan positionierbar ist.

Nach einer Weiterbildung der Erfindung ist das Stellelement als ein Strebenelement ausgebildet, das mehrere Streben umfasst, die eine Öffnung umschließen. Die Öffnung ermöglicht eine direkte Anlage des Heizorgans an dem Temperiergut, wobei die Wärmeübertragungsfläche zwischen dem Heizorgan und dem Temperiergut im Vergleich zu einer Vorrichtung ohne Stellelement nur minimal verringert wird.

Nach einer Weiterbildung der Erfindung weist das Stellelement eine an eine Umfangsfläche des Temperiergutes angepasste Umfangsfläche auf. Durch die Anpassung an die Größenverhältnisse des Temperiergutes kann die Schwenkbewegung mit einem relativ geringen Kraftaufwand herbeigeführt werden.

Nach einer Weiterbildung der Erfindung ist das Stellelement mit einem Aktor, insbesondere einem Schrittmotor gekoppelt, wobei der Aktor das Stellelement derart ansteuert, dass das Stellelement periodisch und/oder nicht-periodisch zwischen einem maximalen und minimalen Stellwinkel hin und her verschwenkt wird. Die Schwenkbewegung weist vorzugsweise eine konstante Amplitude auf. Der Aktor kann statt eines Motors auch als ein magnetisches oder pneumatisches Organ ausgebildet sein. Es ergibt sich hierdurch eine zyklische und homogene Hin- und Herbewegung bzw. Paddelbewegung des Temperiergutes um die Schwenkachse. Alternativ kann diese Bewegung auch mit zeitlich veränderlicher Amplitude ausgeführt sein.

Nach einer Weiterbildung der Erfindung ist das Stellelement derart ansteuerbar, dass es mit einer Frequenz von 0,1 bis 25 Hz fortlaufend verschwenkt wird. Es hat sich herausgestellt, dass in diesem Frequenzbereich die besten Temperierergebnisse erzielt werden. Die Verkürzung der Auftauzeit wird insbesondere durch die stoßartige und an mehreren Stellen des Temperiergutes zyklisch oder antizyklisch partielle Einwirkung einer Stoßkraft erzielt. Hierzu wirkt das Stellelement mit hoher Beschleunigung an unterschiedlichen Stellen des Temperiergutes auf dasselbe ein.

Nach einer Weiterbildung der Erfindung wird das Stellelement derart angesteuert, dass es eine Linearbewegung und/oder Schwenkbewegung mit einer Amplitude im Bereich von +/- 2 mm bis +/- 100 mm, beispielsweise +/- 10 mm bis +/- 30 mm, vorzugsweise +/- 25 mm, ausführt. Es hat sich gezeigt, dass bereits diese relativ kleinen Auslenkungen zu einem guten Temperierergebnis führen.

Nach einer Weiterbildung der Erfindung sind Streben des Strebenelementes aus einem Drahtmaterial gebildet. Vorteilhaft ermöglicht der metallische Draht einen geringen Verlust an Wärmeübertragungsfläche. Infolge der geringen Masse des Drahtmaterials entsteht auch nur eine geringe eigene Wärmekapazität des Strebenelementes. Durch eine steife bzw. stabile Ausgestaltung des Drahtmaterials lassen sich schnelle Bewegungen und hohe Beschleunigungen erzielen, die unmittelbar auf das Temperiergut einwirken. Sofern das Heizorgan als ein Fluidkissen ausgebildet ist, kann nicht nur das Temperiergut, sondern gleichzeitig auch das Fluidkissen durchmischt bzw. stimuliert werden. Das Stellelement kann somit einfach hergestellt werden. Es weist eine für den Einsatzzweck ausreichende Steifigkeit auf.

Nach einer Weiterbildung der Erfindung können entlang einer Schwenkachse auch mehrere Strebenelemente angeordnet sein. Vorteilhaft können hierdurch mehrere kleinere umschlossene Flächen gebildet werden, mittels derer die Schwenkbewegung direkt auf das Temperiergut übertragen wird.

Nach einer Weiterbildung der Erfindung kann das Heizorgan als ein Temperierkissen oder als ein Gelkissen ausgebildet sein, deren Inhalt elektrisch erwärmt wird. Alternativ kann das Heizorgan auch durch einen Kunststoffbeutel enthaltend ein flüssiges, temperiertes Medium ausgebildet sein, wobei das Medium mittels einer Pumpe umgewälzt wird. Das erfindungsgemäße Stellelement kann unabhängig von der Funktionsweise des Heizorganes universell eingesetzt werden.

Ein Ausführungsbeispiel der Erfindung wird nachfolgend anhand der Zeichnungen näher erläutert.

Es zeigen:
- Fig. 1: eine perspektivische Vorderansicht eines Temperiergerätes mit einem aufgeklappten Deckel, wobei auf einem Boden des Gehäuses ein Heizelement und darüber liegend ein mechanisches Stellelement angeordnet sind,

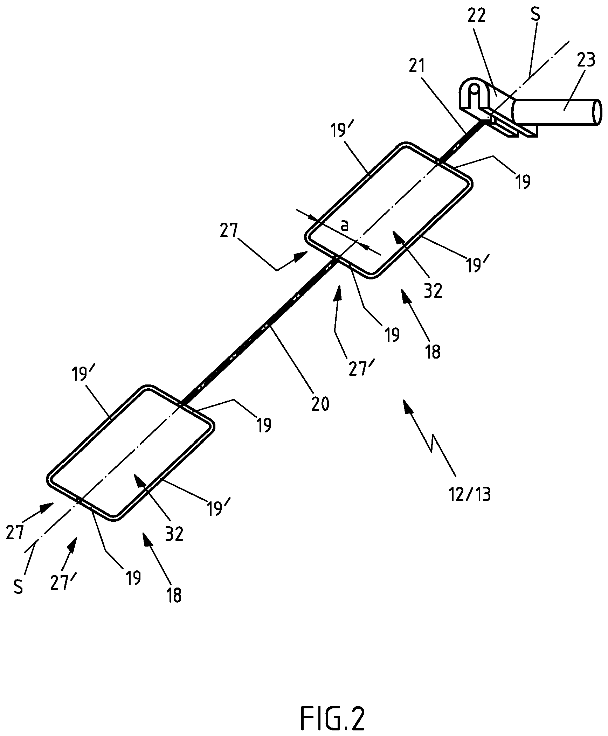

- Fig. 2: eine perspektivische Darstellung eines Stellelementes,
- Fig. 3: eine Explosionsdarstellung der in einer Temperierkammer des Temperiergerätes angeordneten Bauteile im Vertikalschnitt,
- Fig. 4: ein beispielhaftes Zeitdiagramm eines Motors und einer Bewegung der Stellelemente,
- Fig. 5: ein beispielhaftes Zeitdiagramm einer Auslenkung eines Außenbereichs eines Temperiergutes bzw. einer Randstrebe des Stellelementes,
- Fig. 6: eine Draufsicht auf eine Temperierkammer der Temperiervorrichtung, wobei den zwei entlang der Schwenkachse versetzt angeordneten Breitenabschnitten des Stellelementes drei Temperiergüter zugeordnet sind,

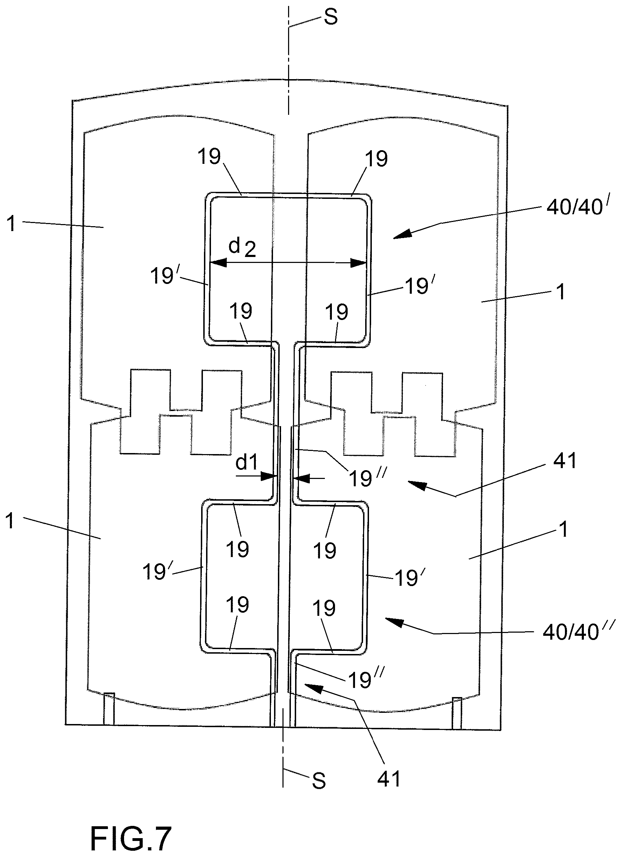

- Fig. 7: eine Draufsicht auf eine Temperierkammer der Temperiervorrichtung, wobei den zwei entlang der Schwenkachse versetzt angeordneten Breitenabschnitten des Stellelementes vier Temperiergüter zugeordnet sind,
- Fig. 8: Weg-/Zeit-Diagramm nach einem ersten beispielhaften Bewegungsprofil der Erfindung,
- Fig. 9: Weg-/Zeit-Diagramm nach einem zweiten beispielhaften Bewegungsprofil der Erfindung und
- Fig. 10: eine Draufsicht auf mehrere Stellelemente.

Eine erfindungsgemäße Vorrichtung zum Temperieren und Auftauen von einem Temperiergut 1 weist ein Gehäuse 2 auf, dessen Oberseite als ein aufklappbarer Deckel 3 ausgebildet ist. Im Innenraum des Gehäuses 2 ist eine untere Basiskammer 4 vorgesehen, innerhalb derer eine elektrische Steuereinheit und andere Baueinheiten zur Ermöglichung einer Temperierung in einer oberhalb der Basiskammer 4 angeordneten Temperierkammer 5 angeordnet sind. In Höhe der Basiskammer 4 weist das Gehäuse 2 vorderseitig Bedienelemente 6 und eine Anzeigeeinheit 7 auf, so dass das von dem Gehäuse 2 umfasste Auftaugerät von einer Person bedienbar ist.

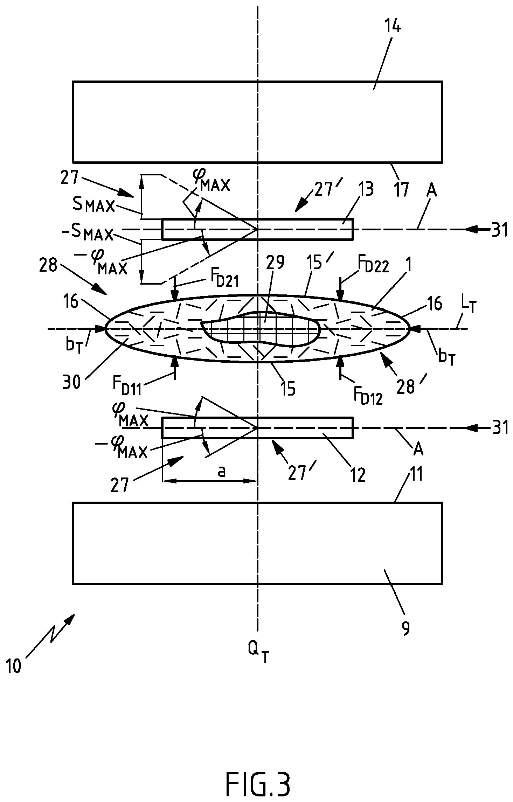

Die Temperierkammer 5 weist einen festen Boden 8 auf, der zugleich eine Trennwand bildet zu der Basiskammer 4. Das Heizorgan besteht u.a. aus dem auf dem Boden 8 befindlichen, ersten Heizelement 9 sowie einem dazu gehörigen, in der Basiskammer 4 angeordneten Heizungsmodul 10. In Höhe einer Oberseite 11 des unteren Heizelementes 9 ist ein mechanisches Stellelement 12 angeordnet. Im Betriebszustand des Auftaugerätes, in dem der Deckel 3 geschlossen ist, weist die Temperierkammer 5 den in Figur 3 dargestellten, schichtweisen Aufbau in der Höhe auf. In vertikaler Richtung nach oben schließt sich an das erste Stellelement 12 das Temperiergut 1 an. Oberhalb des Temperiergutes 1 schließt sich ein zweites oberes Stellelement 13 an. Oberhalb des zweiten Stellelementes 13 schließt sich ein zweites Heizelement 14 des Heizmoduls 10 an. Bezüglich einer Längsmittelebene L_{T} des Temperiergutes 1 weist die Temperierkammer 5 einen idealerweise symmetrischen Aufbau auf. Zu beiden Seiten des Temperiergutes 1 ist ein Stellelement 12, 13 und daran anschließend ein Heizelement 9, 14 angeordnet.

Das Temperiergut 1 weist beispielsweise ein Plasma- oder Blutmaterial auf, das von einem Beutel als Umverpackung umgeben ist. Idealerweise sind die Beutel des Temperiergutes 1 relativ flach ausgebildet, so dass das Temperiergut 1 zwei gegenüberliegende Seiten 15, 15' und umlaufende Schmalseiten 16 aufweist. Die Schmalseiten 16 verbinden die gegenüberliegenden Seiten 15, 15'. Die Seiten 15, 15' sind vorzugsweise rechteckförmig ausgebildet.

Alternativ kann das Temperiergut 1 auch polygonartig mit unterschiedlichen oder gleichgroßen Seiten ausgebildet sein. Beispielsweise kann das Temperiergut 1 auch birnenförmig oder würfelförmig oder klumpenförmig ausgebildet sein. Das Temperiergut 1 wird hierbei in die Temperierkammer 5 eingesetzt, wobei es von den Stellelementen 12, 13 und/oder den Heizelementen 9, 14 eingeklemmt wird. Aufgrund der vorzugsweise nachgiebigen Ausgestaltung des Temperiergutes, zumindest wenn es sich in einem fortgeschrittenen aufgetauten Zustand befindet, können sich Flachseiten bilden, so dass die Wärmeeintragungsfläche im Vergleich zum Ausgangszustand erhöht ist.

Zur besseren Darstellbarkeit sind die Bauteile in Figur 3 beabstandet zueinander angeordnet. Tatsächlich liegen sie dicht übereinander an. Somit liegt das erste untere Stellelement 12 direkt und flächig an der unteren Seite 15 des Temperiergutes 1 und an der Oberseite 11 des ersten unteren Heizelementes 9 an. Das obere zweite Stellelement 13 liegt direkt und flächig an der oberen Seite 15' des Temperiergutes 1 und an einer Unterseite 17 des oberen zweiten Heizelementes 14 an.

Das erste Stellelement 12 und das zweite Stellelement 13 können vorzugsweise baugleich ausgebildet sein. Sie weisen jeweils zwei in Längsrichtung des Stellelementes 12, 13 verteilt angeordnete Paddelabschnitte 18 auf, die jeweils aus rechteckförmig verlaufenden Streben 19 bestehen. Die Paddelabschnitte 18 sind durch eine Verbindungsstrebe 20 miteinander verbunden. Die Verbindungsstrebe 20 kann durchgehend von einem ersten Ende zu einem zweiten Ende des Stellelementes 12, 13 verlaufen. An einem Ende der Verbindungsstrebe 20 schließt sich ein T-Stück 21 an, das in einer Aufnahme 22 beispielsweise rastend gelagert ist. Die Aufnahme 22 weist hierfür eine Nut auf, in der eine endseitige Querstrebe des T-Stücks 21 gelagert ist. An die Aufnahme 22 schließt sich ein Hohlzylinder 23 an, in dem eine nicht dargestellte Welle eines Motors drehfest eingreifbar ist. In Betriebsstellung verläuft der Hohlzylinder 23 bzw. die Welle koaxial zu der Verbindungsstrebe 20, die somit in einer Schwenkachse S verläuft. Der Motor dient als Aktor. Alternativ kann der Aktor auch pneumatisch oder magnetisch ausgebildet sein, beispielsweise als Hubmagnet oder als Drehmagnete.

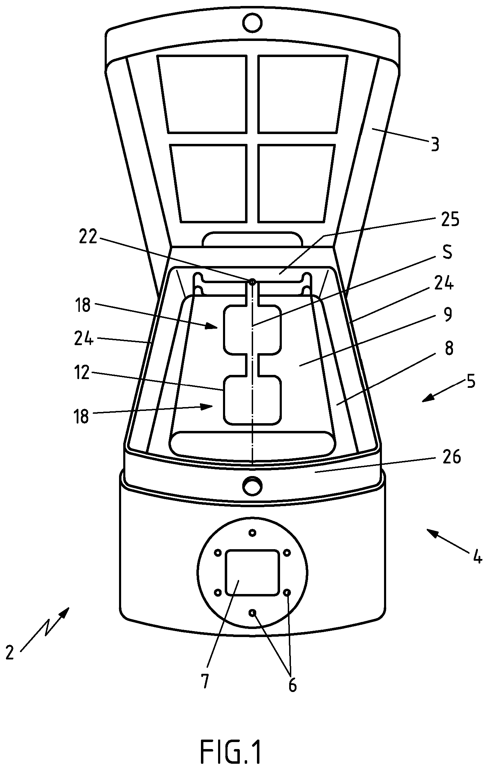

Das erste Stellelement 12 und das zweite Stellelement 13 sind mittels des Motors, mit dem sie über die Welle direkt oder über ein Getriebe gekoppelt sind, periodisch und/oder nicht-periodisch zwischen einem maximalen und einem minimalen Stellwinkel φ_{MAX}, -φ_{MAX} schwenkbar. Wie aus Figur 1 ersichtlich ist, sind die Stellelemente 12, 13 zentral in unterschiedlichen Höhen der Temperierkammer 5 gelagert. Die Schwenkachsen S verlaufen üblicherweise parallel zu einer Seitenwand 24 der Temperierkammer 5 sowie senkrecht zu einer Rückwand 25 sowie einer Vorderwand 26 der Temperierkammer 5. Im Bereich der Rückwand 25 ist die Aufnahme 22 der Stellelemente 12, 13 angeordnet.

Die Schwenkachsen S, um die die Stellelemente 12, 13 hin und her verschwenkbar gelagert sind, verlaufen entlang oder nah einer Mittelebene, nämlich einer Quermittelebene Q_{T}, des Temperiergutes 1. Eine erste seitliche Hälfte 27 des Paddelabschnitts 18 der Stellelemente 12, 13 ist somit einer ersten Hälfte 28 des Temperiergutes 1 zugeordnet. Eine zweite Hälfte 27' der Paddelabschnitte 18 der Stellelemente 12, 13 ist einer zweiten Hälfte 28' des Temperiergutes 1 zugeordnet. Beide Hälften 27, 27' bzw. 28, 28' können symmetrisch zu der Schwenkachse S bzw. Quermittelebene Q_{T} angeordnet sein.

Die in Richtung der Schwenkachse S versetzt angeordneten Stellelemente 12, 13 werden vorzugsweise gleich angesteuert, so dass während einer halben Periodendauer T/2 die ersten Hälften 27 der Paddelabschnitte 18 um einen positiven Winkel φ in Richtung +φ_{MAX} und die zweiten Hälften 27' um einen negativen Winkel -φ in Richtung -φ_{MAX} verdreht werden. Während die ersten Hälften 27 der Stellelemente 12, 13 nach oben verschwenkt werden, werden hierbei die zweiten Hälften 27' derselben nach unten verschwenkt. Auf das Temperiergut 1 wird somit an gegenüberliegenden Seiten 15, 15' und an gegenüberliegenden Hälften 28, 28' eine Druckkraft F_{D} ausgeübt. Hierdurch können gezielt inhomogene Strömungen in dem Temperiergut 1 erzeugt werden, wobei sich beispielsweise ein gefrorener Kern 29 hin und her bewegt und von inhomogenen Strömungen der bereits aufgetauten Flüssigkeit 30 umgeben ist.

Hierdurch kann ein besonders hoher Temperiergradient erzeugt werden. Der gefrorene Kern 29 ist ein Teil des Temperiergutes 1, der sich in einem Kernbereich des Temperiergutes 1 befindet. Die den Kern 29 umgebende Flüssigkeit 30 entspricht einem Teil des Temperiergutes 1, der sich in einem Außenbereich des Temperiergutes 1 befindet. Das Temperiergut 1 befindet sich zu Beginn des Temperiervorganges in einem gefrorenen Zustand, wobei sowohl der Außenbereich als auch der Kernbereich des Temperiergutes sich in einem festen Aggregatzustand befinden, also eine Temperatur von 0°C oder niedriger aufweisen. Durch Beaufschlagung des Temperiergutes 1 durch die erfindungsgemäße Vorrichtung wird relativ schnell unter Auftauen des Außenbereiches des Temperiergutes 1 und durch schnelle Bewegung unter hoher Beschleunigung des Temperiergutes 1 dasselbe vollständig aufgetaut, bis es am Ende des Temperiervorganges die gewünschte Temperatur aufweist.

Wie aus Figur 3 ersichtlich ist, schneiden die Schwenkachsen S der Stellelemente 12, 13 in orthogonaler Projektion auf eine Längsmittelebene L_{T} des Temperiergutes 1 dasselbe.

Die Paddelabschnitte 18 der Stellelemente 12, 13 weisen üblicherweise eine Umfangsfläche auf, die kleiner ist als eine Umfangsfläche des Temperiergutes 1.

Nach einer alternativen Ausführungsform der Erfindung können den Paddelabschnitten auch mehrere Temperiergüter 1 zugeordnet sein.

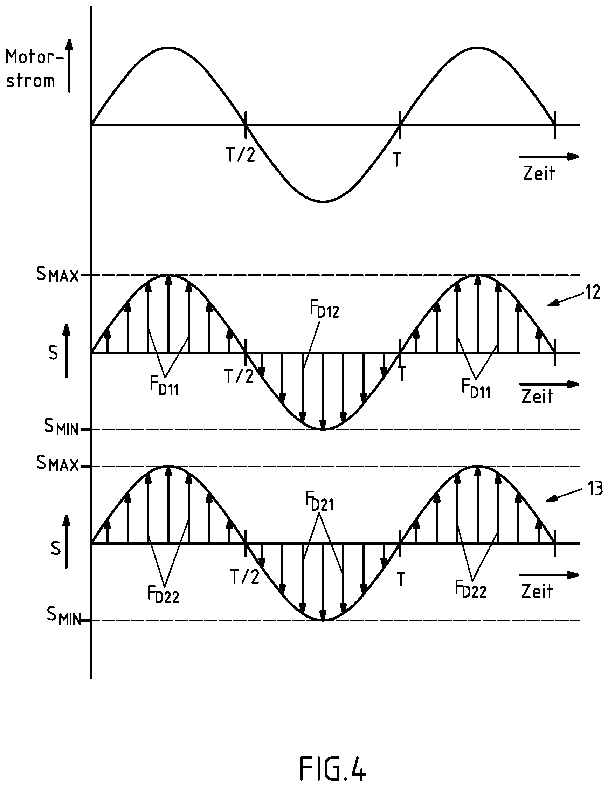

Wie aus Figur 4 ersichtlich ist, werden die Paddelabschnitte 18 um die Schwenkachse S im Beispiel derart periodisch verschwenkt, dass eine parallel zu der Schwenkachse S verlaufende Randstrebe 19' zyklisch eine maximale Auslenkung +/- s_{MAX} bezogen auf eine Ausgangslage bzw. Ausgangsniveau 31 durchläuft. Die Ausgangslage 31 verläuft in einer zu der Längsmittelebene L_{T} des Temperiergutes 1 parallelen Ebene. Die maximale Auslenkung S_{MAX}, -S_{MAX} kann im Bereich von +/- 30 mm, vorzugsweise im Bereich von +/- 25 mm, liegen.

Die Randstreben 19' weisen jeweils einen Abstand a zu der Schwenkachse S auf, der 0,2 bis 0,7 einer hälftigen Breite b_{T} des Temperiergutes 1 entspricht.

Im vorliegenden Ausführungsbeispiel ist das mechanische Stellelement 12, 13 bzw. der Paddelabschnitt 18 eben ausgebildet.

Nach einer nicht dargestellten alternativen Ausführungsform der Erfindung kann die Kontur des Stellelementes 12, 13 bzw. der Paddelabschnitte 18 auch z.B. bogenförmig und/oder löffelförmig sein, um besser an die Form des Temperiergutes 1 angepasst zu sein.

Die Streben 19, 19' der Paddelabschnitte 18 begrenzen eine Öffnung 32, in die das flexibel ausgebildete Temperiergut 1 und/oder das Heizelement 9, 14 teilweise eingreifen kann. Es entsteht somit im Bereich der Öffnung ein direkter Kontakt des Temperiergutes 1 mit dem Heizelement 9, 14. Da das Stellelement 12, 13 nach der vorliegenden Ausführungsform insgesamt als Strebenelement ausgebildet ist, kann im Wesentlichen eine direkte Kontaktierung der Heizelemente 9, 14 mit dem Temperiergut 1 verwirklicht werden.

Nach einer alternativen Ausführungsform der Erfindung kann der Paddelabschnitt 18 auch vollflächig aus einem wärmeleitenden Material steif oder flexibel ausgebildet sein. Gegebenenfalls kann das vollflächige Stelleelement bzw. Paddelabschnitt 18 aus mehreren steifen Segmenten bestehen, die beispielsweise nach Art eines Filmscharniers miteinander verbunden sind.

Der Motor verschwenkt das Stellelement 12, 13 vorzugsweise mit einer Frequenz im Bereich von 0,5 bis 5 Hz. Mit dieser Frequenz kann ein optimaler Wärmeeintrag in das Temperiergut 1 erfolgen.

Die Heizelemente 9, 14 weisen jeweils einen Kunststoffbeutel auf, in dem ein flüssiges, temperiertes Medium mittels einer nicht dargestellten Pumpe umgewälzt wird. Die Pumpe und Heizschlangen zur Temperierung des flüssigen Mediums sind in der Basiskammer 4 angeordnet.

Nach einer alternativen Ausführungsform der Erfindung können die Heizelemente 9, 14 auch durch Temperierkissen oder Gelkissen gebildet sein, die jeweils elektrisch erwärmt werden. Der Bauraumaufwand ist hierdurch vergleichsweise gering.

Die Streben 19, 19' des Stellelementes 12, 13 bestehen aus einem Drahtmaterial.

Nach einer nicht dargestellten alternativen Ausführungsform der Erfindung kann das Auftaugerät auch lediglich ein einziges Stellelement 12, 13 aufweisen, das entweder oberhalb oder unterhalb des Temperiergutes 1 angeordnet ist. Im Unterschied zu der beschriebenen Ausführungsform wird nicht ständig an beiden Seiten 15, 15' des Temperiergutes 1 eine Druckkraft F_{D} angewandt, sondern nur abwechselnd an einer einzigen Seite 15, 15', nämlich während einer ersten Halbperiode T/2 auf der ersten Seite 15 und während der zweiten Halbperiode T/2 auf der zweiten Seite 15'.

Nach einer nicht dargestellten Ausführungsform kann der Motor auch als Schrittmotor ausgebildet sein, mittels dessen die Welle um einen vorgegebenen Winkel verdreht wird. Die in Figur 4 dargestellte Motorkurve mit dem Motorstrom I wird in diesem Fall keinen sinusförmigen Verlauf aufweisen.

Wie in Zusammenschau von Figur 3 und Figur 4 ersichtlich ist, werden beispielhaft periodisch Druckkräfte F_{D} auf beide Seiten des Temperiergutes 1, also auf die erste Seite 15 und die zweite Seite 15' ausgeübt. Es wird davon ausgegangen, dass die Stellelemente 12, 13 synchron und/oder gleichsinnig in die gleichen Schwenkrichtungen verschwenkt werden. Somit wird beim nach oben Schwenken der ersten Hälfte 27 der Paddelabschnitte 18 aus der Ausgangslage in der Ebene A nach oben auf das Temperiergut 1 eine Drucckraft F_{D11} von dem unteren Stellelement 12 und beim nach unten Schwenken der zweiten Hälfte 27' der Paddelabschnitte 18 auf das Temperiergut 1 eine entgegengesetzt gerichtete Druckkraft F_{D22} von dem oberen Stellelement 13 ausgeübt. Haben die Stellelemente 12, 13 ihre maximale Auslenkung S_{MAX} unter Bildung des maximalen Winkels φ_{MAX} zu der Ebene A der Ausgangslage erreicht, erfolgt eine Bewegungsumkehr, so dass die Druckkräfte F_{D11}, F_{D22} nachlassen. Mit Erreichen der Ausgangslage A nach einer Halbperiodendauer T/2 wirkt nun von den anderen Hälften 27' des unteren Stellelementes 12 eine Druckkraft F_{D12} auf die zweite Hälfte 27' der unteren Seite 15, während von einer ersten Hälfte 27 des oberen Stellelementes 13 eine Druckkraft F_{D21} auf die erste Hälfte 27 des Temperiergutes 1 von oben wirkt. In Figur 4 ist dies durch entsprechende Kraftpfeile verdeutlicht. Die Hälften 27, 27' der Paddelabschnitte 18 wirken somit wie Schwenkhebel, die Öffnungen aufweisen. Es wird ständig eine Druckkraft F_{D} auf die untere Seite 15 und die obere Seite 15' des Temperiergutes 1 ausgeübt, wobei die Druckkräfte F_{D11} und F_{D22} bzw. F_{D21} und F_{D12} bezüglich der Quermittelebene Q_{T} asymmetrisch auf das Temperiergut 1 einwirken

Wenn lediglich ein einziges Stellelement 12, 13 vorgesehen ist, erfolgen die Druckkräfte F_{D11}, F_{D12} oder F_{D21}, F_{D22} lediglich von einer einzigen Seite her abwechselnd von den Hälften 27, 27' der Paddelabschnitte 18 auf beide Hälften 28, 28' der Seite 15 oder 15' des Temperiergutes 1.

Nach einer nicht dargestellten alternativen Ausführungsform der Erfindung könnten die beiden Stellelemente 12, 13 auch so angesteuert werden, dass sie nicht gleichsinnig, sondern gegensinnig (antizyklisch) verschwenkt werden, so dass abwechselnd in den Hälften 28 der unteren Seite 15 und der oberen Seite 15' des Temperiergutes 1 einerseits und in den Hälften 28' der unteren Seite 15 und der oberen Seite 15' des Temperiergutes 1 eine Druckkraft F_{D} erzeugt wird. Während die erste Hälfte 27 des ersten Stellelements 12 nach oben verschwenkt wird, wird die erste Hälfte 27 des zweiten Stellelements 13 nach unten verschwenkt. Während die zweite Hälfte 27' des ersten Stellelements 12 nach unten verschwenkt wird, wird die zweite Hälfte 27' des zweiten Stellelements 13 nach oben verschwenkt. Durch diese gegenförmige bzw. gegensinnige Bewegung der Stellelemente 12, 13 erfolgt ein intensiveres Bewegen des gefrorenen Kerns 29 sowie der bereits aufgetauten Bestandteile des Temperiergutes 1. Vorteilhaft kann hierbei im Vergleich zu der Ausführungsform mit einem einzigen Stellelement die gleiche Wirkung mit einem verringerten maximalen Schwenkwinkel φ_{MAX}, -φ_{MAX} bzw. maximale Auslenkung S_{MAX}, -S_{MAX} erreicht werden.

Alternativ können die Stellelemente 12, 13 auch periodisch und/oder nicht-periodisch gleichsinnig oder gegensinnig zueinander bewegt werden, so dass die Verformungskraft auf das Temperiergut 1 bzw. seinen Kern 29 sowie seine bereits aufgetaute umgebende Flüssigkeit 30 weiter erhöht wird.

Nach einer nicht dargestellten Ausführungsform kann statt einer periodischen Bewegung eine nicht-periodische Bewegung oder eine nicht-periodische Schwenkbewegung des mindestens einen Stellelements 12, 13 vorgesehen sein.

Nach einer nicht dargestellten Ausführungsform kann eine zeitliche Abfolge von einer periodischen Bewegung und einer nicht-periodischen Bewegung des mindestens einen Stellelements 12, 13 vorgesehen sein.

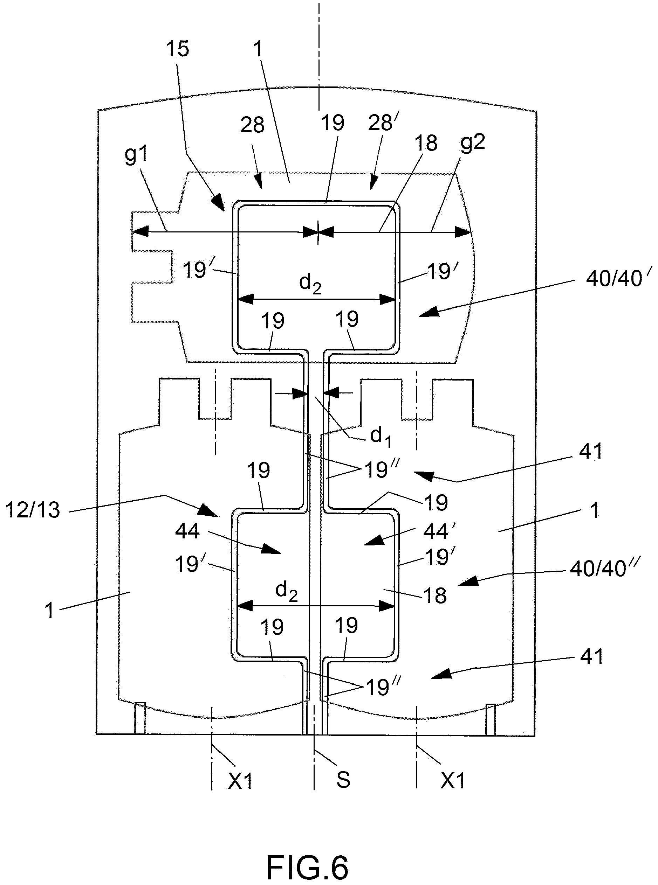

Nach einer Ausführungsform des Stellelementes gemäß Figur 6, die auch in Figur 1 dargestellt ist, besteht das Stellelement 12, 13 aus mehreren entlang der Schwenkachse S versetzt zueinander angeordneten Breitenabschnitten 40 und Schmalabschnitten 41. Der Breitenabschnitt 40 des Stellelementes 12, 13 umfasst ein Paar von in einem relativ großen Abstand d2 verlaufenden Randstreben 19'. Die Randstreben 19' verlaufen parallel zur Schwenkachse S. An den Randstreben 19' schließen sich Streben 19 an, die im Wesentlichen senkrecht zur Schwenkachse S verlaufen. Die Randstreben 19' und die benachbarten Streben 19 bilden den O-förmigen Paddelabschnitt 18. Der O-förmige Paddelabschnitt 18 ist als ein offenes Paddel ausgeführt, das eine Öffnung aufweist.

In Richtung der Schwenkachse S schließt sich an dem Breitenabschnitt 40 der Schmalabschnitt 41 mit parallel zur Schwenkachse S verlaufenden Streben 19" an, die in einem relativ kleinen Abstand d1 zueinander angeordnet sind. Die Streben 19" des Schmalabschnitts 41 verlaufen in Richtung der Schwenkachse. Die Streben 19" des Schmalabschnitts 41 schließen sich an den quer zur Schwenkachse verlaufenden Streben 19 des Breitenabschnitts 40 an oder sind an einem Ende des Stellelementes 12, 13 mit dem Aktor gekoppelt.

Wie aus Figur 6 zu ersehen ist, liegt ein erster Breitenabschnitt 40' an den Seiten 15, 15' eines quer zur Schwenkachse S angeordneten Temperiergutes 1 an. Der erste Breitenabschnitt 40' ist an das Temperiergut 1 derart angepasst, dass die Randstreben 19' im Bereich von +/- 20 %, vorzugsweise von +/- 10% oder alternativ in der Nähe eines Massenschwerpunktes oder auf dem Massenschwerpunkt der Hälften 28, 28' des Temperiergutes 1 verlaufen. Im vorliegenden Ausführungsbeispiel beträgt ein Abstand g1 der ersten Hälfte 28 8 cm und ein Abstand g2 der zweiten Hälfte 28' 7 cm. Der Abstand d2 der Randstreben 19' ist somit so groß gewählt, dass das Temperiergut 1 um die in einer Quermittelebene Q_{T} desselben verlaufende Schwenkachse S mit angemessenem Kraftaufwand ausgelenkt werden kann. Die durch die Randstreben 19' bewirkte Angriffslinie in Richtung der Schwenkachse S an den Hälften 28, 28' verläuft somit im Bereich des Massenschwerpunktes dieser Hälften 28, 28'.

Ein zweiter Breitenabschnitt 40" des Stellelementes 12, 13 greift an zwei benachbart zueinander angeordneten Temperiergütern 1 an. Sämtliche Temperiergüter 1 sind in einer gemeinsamen Ebene angeordnet. Eine erste Hälfte 44 des Breitenabschnitts 40" ist dem zweiten Temperiergut 1 und eine zweite Hälfte 44' des Breitenabschnitts 40" dem dritten Temperiergut 1 zugeordnet. Die Hälften 44, 44' des Breitenabschnitts 40" sind symmetrisch zu der Schwenkachse S angeordnet. Die Randstreben 19' der Hälften 44, 44' weisen jeweils den gleichen halben Abstand d2/2 zu der Schwenkachse S auf. Die Randstreben 19' verlaufen im Bereich eines Massenschwerpunktes der jeweiligen Temperiergüter 1 bzw. in der Nähe oder im Bereich einer Symmetrieachse X1 der Temperiergüter 1.

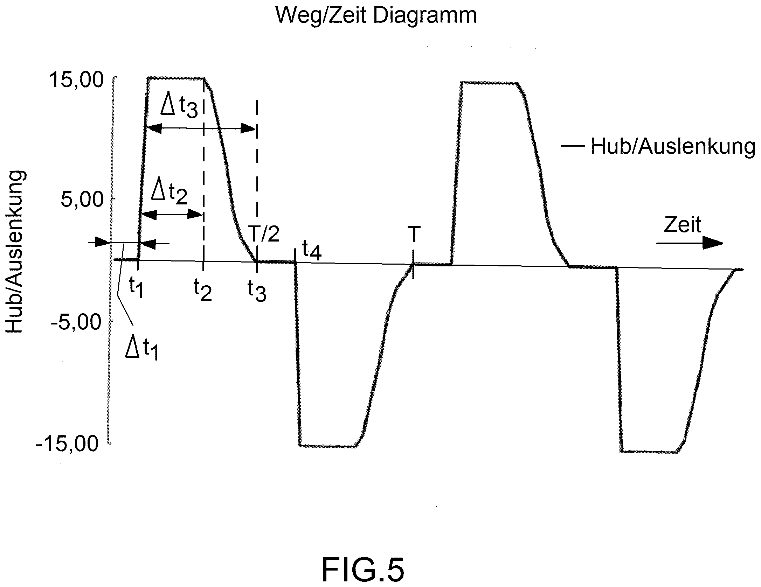

Nach einer weiteren Ausführungsform der Erfindung gemäß Figur 5 kann statt einer kontinuierlichen Auslenkung der Stelleelemente 12, 13 (harmonische und/oder lineare Schwingung) eine impulsartige Auslenkung erfolgen. Die Randstrebe 19' kann beispielsweise zum Zeitpunkt t₁ schlagartig von 0 cm auf 15 cm ausgelenkt werden. In einem Zeitintervall von Δt2 verharrt das Stellelement 12, 13 dann in Ruhe, bevor es zum Zeitpunkt t2 schlagartig, aber mit geringerer betragsmäßiger Beschleunigung als zur Auslenkbewegung, wieder zurück in die Ausgangsstellung verbracht wird. Nach Erreichung der Ausgangsstellung zum Zeitpunkt t3 verharrt das Stelleelement 12, 13 bis zum Zeitpunkt t4 in Ruhe, bevor die gleiche Auslenkbewegung in die andere Schwenkrichtung erfolgt. Nach dieser Ausführungsform sind stoßartige periodische und/oder nicht-periodische Auslenkungen vorgesehen, wobei sich das Stellelement 12, 13 in der maximalen Auslenklage im Zeitintervall Δt2 und in der Ausgangslage im Zeitintervall Δt1 in Ruhe befindet.

Die maximale Auslenkung und/oder die Frequenz 1/T und/oder die Beschleunigung der Bewegung des Stellelementes 12, 13 kann in Abhängigkeit der aktuellen Temperatur des Temperiergutes 1 in Verbindung mit einer vorgegebenen Schwelltemperatur oder einem fest vorgegebenen Zeitpunkt gewählt werden. Die Ansteuerung der Stellelemente 12, 13 muss nicht über die gesamte Laufzeit des Temperiervorganges periodisch oder nicht-periodisch oder mit der gleichen Impulsfolge oder mit dem gleichen Auslenkverlauf erfolgen. Die Höhe der Auslenkung bzw. die Frequenz sowie die Beschleunigung kann in Abhängigkeit von dem zu temperierenden Temperiergut 1 verändert werden. Es können somit unterschiedliche Bewegungsprofile für unterschiedliche Temperiergüter 1 gewählt werden. Beispielsweise kann zu Beginn des Temperiervorganges die Frequenz der impulsartigen Auslenkungen erhöht sein, bis der gefrorene Kern 29 des Temperiergutes 1 auf ein Mindestvolumen geschrumpft ist. Im Anschluss daran kann die Frequenz der Impulsfolge reduziert werden. Alternativ kann auch in einem ersten Abschnitt des Temperiervorganges die maximale Auslenkung relativ klein gewählt werden, bis der Außenbereich des Temperiergutes aufgetaut ist und sich im flüssigen Zustand befindet. Im zweiten Teil des Temperiervorganges kann dann die maximale Auslenkung des Stellelementes 12, 13 erhöht werden, so dass die Durchmischung innerhalb des Temperiergutes erhöht und damit der Auftauvorgang beschleunigt werden kann. Im weiteren Verlauf des Temperiervorganges kann dann die maximale Auslenkung und/oder die Frequenz und/oder die Beschleunigung wieder verringert werden, bis das Temperiergut 1 die gewünschte Solltemperatur eingenommen hat.

Zur Ansteuerung des Stellelementes 12, 13 kann statt eines Stellmotors auch ein Schrittmotor oder ein Gleichstrom-/Wechselstrommotor mit Getriebe oder ein elektrisch betriebener Hub-/Drehmagnet als Aktor eingesetzt werden. Alternativ kann zur Ansteuerung des Stellelementes 12, 13 auch ein pneumatisch oder hydraulisch betriebener Zylinder eingesetzt werden.

Nach einer alternativen Ausführungsform der Erfindung kann statt einem gefrorenen Temperiergut auch ein beliebiger chemischer Stoff oder beliebiges Material, das sich im flüssigen oder zähflüssigen Zustand befindet, auf eine gewünschte Temperatur verbracht werden.

Wenn das Temperiergut aus einem relativ großflächigen bzw. großvolumigen Material, wie beispielsweise Beton oder dergleichen, besteht, erfolgt die Auslenkung des Temperiergutes an mehreren Stellen von vorzugsweise gegenüberliegenden Seiten ausschließlich über Stellelemente, die linear bewegt werden. Es ergeben sich somit vorzugsweise mehrere Stoßpunkte an dem Temperiergut, an denen das Stellelement in gemeinsamer Stellrichtung oder parallel versetzt zueinander angreifen. Insbesondere können hierbei die Stellelemente kaskadiert quer versetzt zur Stellrichtung derselben angeordnet sein, wobei die Stellelemente quer zur Stellrichtung zeitversetzt angesteuert bzw. auf das Temperiergut einwirken können.

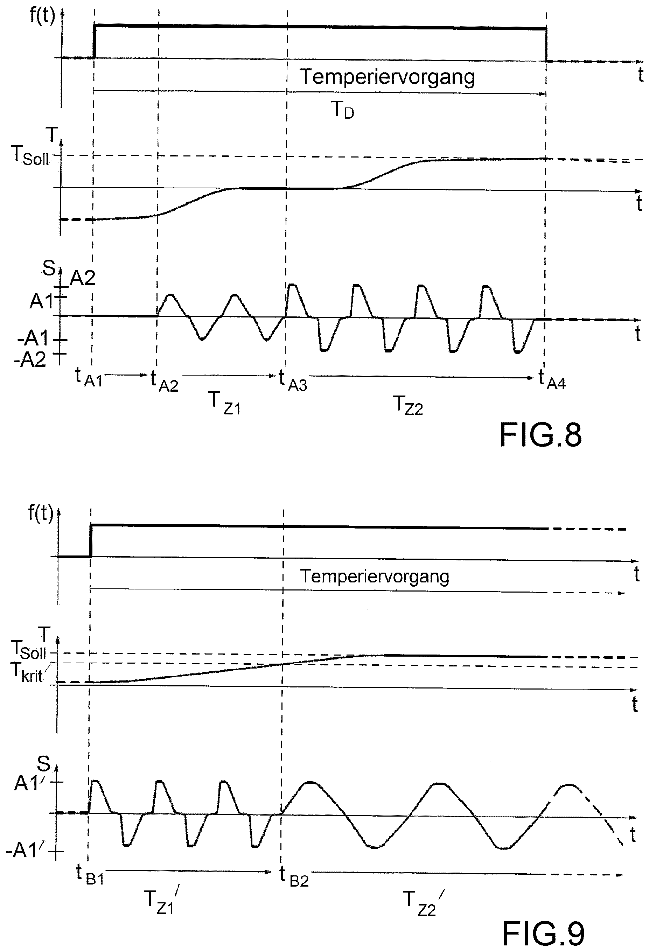

In Figur 8 ist ein Temperiervorgang gemäß einem ersten Bewegungsprofil dargestellt. Grundsätzlich kann in Abhängigkeit von Betriebs- oder Prozessparametern mittels des Stellelementes 12, 13 partiell eine Druckkraft auf das Temperiergut 1 ausgeübt werden. Eine Einwirkdauer, eine Einwirkgröße und/oder eine Einwirkstärke (Beschleunigung) des Stellelementes 12, 13 kann innerhalb des Temperiervorganges variiert werden.

In Figur 8 ist ein Bewegungsprofil zum Auftauen eines Temperiergutes (Plasma) aus einem gefrorenen Zustand in einen flüssigen Zustand einer vorgegebenen Solltemperatur T_{Soll} dargestellt. In einem Vorlaufzeitintervall zwischen den Zeitpunkten t_{A1} und t_{A2} befindet sich das Temperiergut 1 noch nahezu vollständig im gefrorenen Ausgangszustand und es findet noch keine Bewegung statt. In einem ersten Zeitintervall T_{Z1} zwischen den Zeitpunkten t_{A2} und t_{A3} ist bereits ein Teil des Temperiergutes verflüssigt und es wird das Stellelement 12, 13 beispielhaft periodisch mit einer ersten Frequenz und einer ersten, reduzierten Amplitude sowie einem ersten betragsmäßigen Anstieg (Beschleunigung) betrieben, so dass die Temperatur des Temperiergutes 1 in die Nähe von 0°C gelangt. Das Zeitintervall T_{Z2} beginnt zum Zeitpunkt t_{A3} und endet zum Zeitpunkt t_{A4}. Im Vergleich zum ersten Zeitintervall T_{Z1} wird das Stellelement 12, 13 mit einer größeren Amplitude A2 und einem betragsmäßig größeren zweiten Anstieg der Auslenkung (Beschleunigung) zu der Amplitude A2, jedoch mit gleicher Frequenz beispielhaft periodisch bewegt. Die geänderte Ansteuerung des Stellelementes 12, 13 erfolgt zu einem fest vorgegebenen Zeitpunkt t_{A3} oder in Abhängigkeit von einem Betriebs- oder Prozessparameter, beispielsweise der Laständerung des Stellelementes 12, 13, was durch einen veränderten Motorstrom des Aktors erkannt werden kann. Unter dem Anstieg zur Amplitude des Stellelementes 12, 13 wird die Steigung des Wegverlaufs/Auslenkung bzw. die Beschleunigung des Stellelementes 12, 13 verstanden, die sich von dem Nullpunkt bis zur Amplitude A1, A2 erstreckt. Wie aus dem zweiten Zeitintervall T_{Z2} zu ersehen ist, ist der Betrag des Anstiegs der Auslenkung zu der Amplitude A2 bzw. -A2 am größten, während die Rückbewegung zur Nulllinie mit einem geringeren betragsmäßigen Anstieg bzw. geringerer betragsmäßiger Beschleunigung erfolgt.

Nach einer weiteren Ausführungsform eines Bewegungsprofils gemäß Figur 9 erfolgt die Ansteuerung des Stellelementes 12, 13 bezüglich eines Temperiergutes 1, das sich in einem gekühlten, jedoch nicht gefrorenen Zustand befindet. Das Temperiergut 1 kann als ein chemischer Zusatzstoff ausgebildet sein. Das Temperiergut 1 kann sich somit zu Beginn des Temperiervorganges im flüssigen oder zähflüssigen Zustand befinden. Das Temperiergut 1 soll auf eine Solltemperatur T_{Soll} erwärmt werden. Hierzu wird in einem ersten Zeitintervall T_{Z1}', das zum Zeitpunkt t_{B1} beginnt und zum Zeitpunkt t_{B2} endet, das Stellelement beispielhaft periodisch mit einer ersten Frequenz, einer ersten Amplitude sowie einem betragsmäßigen ersten Anstieg (Beschleunigung) zur maximalen Auslenkung A1', -A1' bewegt. Die Änderung der Bewegung des Stellelementes 12, 13 erfolgt bei Erreichen einer kritischen Temperatur Tₖᵣᵢₜ, in der das Temperiergut 1 zu einem unerwünschten Aufschäumen neigt. In dem nun beginnenden zweiten Zeitintervall T_{Z2}' erfolgt die Bewegung des Stellelementes 12, 13 mit der gleichen Amplitude A1, -A1', jedoch mit einer geringeren Frequenz sowie einer geringeren Beschleunigung bzw. einem geringeren betragsmäßigen Anstieg zur maximalen Auslenkung A1', -A1'.

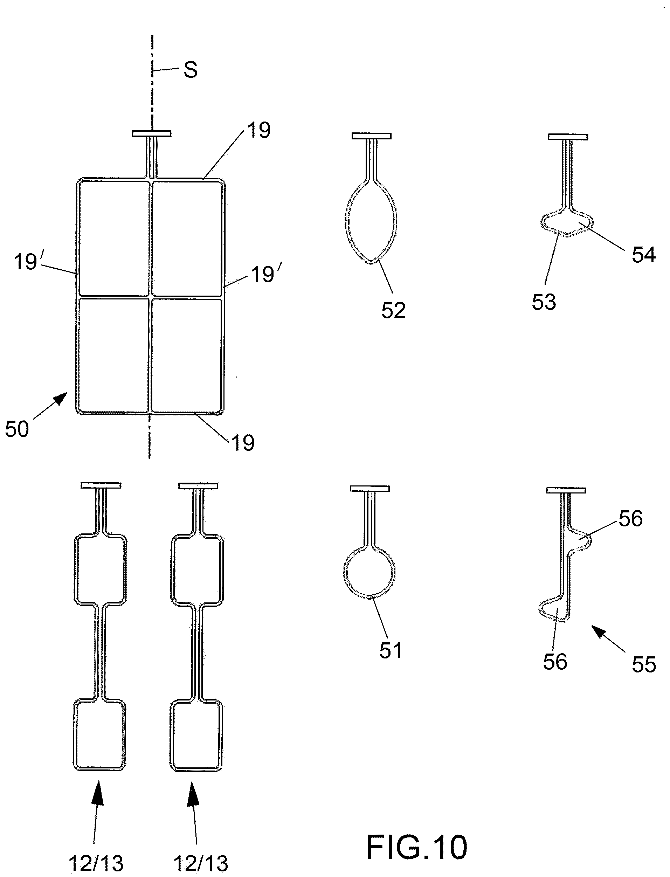

Wie aus Figur 10 zu ersehen ist, können die beispielsweise in Figur 6 dargestellten Stellelemente 12, 13 paarweise nebeneinander in einem vorgegebenen Abstand zueinander innerhalb der Temperierkammer 5 angeordnet sein, s. links unten in Figur 10. Alternativ kann in der Temperierkammer 5 auch ein einziges Stellelement 50 angeordnet sein, das rechteckförmige Streben 19 mit symmetrisch zur Schwenkachse S verlaufende Randstreben 19' aufweist. Die Randstreben 19' verlaufen vorzugsweise durchgehend und geradlinig über die gesamte Länge des Stellelementes 50.

Nach einer alternativen Ausführungsform kann das Stellelement auch kreisrunde Streben 51 (kreisförmiger Paddelabschnitt), s. Figur 10 in der Mitte unten, oder ellipsenförmige Streben 52 (ellipsenförmiger Paddelabschnitt), s. Figur 10 oben in der Mitte, aufweisen.

Nach einer weiteren Ausführungsform kann ein Stellelement 53 vorgesehen sein, das einen rautenförmigen Paddelabschnitt 54 aufweist, s. Figur 10 oben rechts.

Nach einer weiteren Ausführungsform kann ein Stellelement 55 vorgesehen sein, das bezüglich der Schwenkachse S asymmetrisch angeordnete Paddelabschnitte 56 aufweist.

In Abhängigkeit von der Dimension des Temperiergutes 1 können somit unterschiedlich geformte Stellelemente 12, 13, 50, 51, 53, 55 eingesetzt werden.

Es versteht sich, dass die vorstehend genannten Merkmale je für sich oder zu mehreren in beliebiger Kombination Verwendung finden können. Die beschriebenen Ausführungsbeispiele sind nicht als abschließende Aufzählung zu verstehen, sondern haben vielmehr beispielhaften Charakter für die Schilderung der Erfindung.

### Bezugszeichenliste

- 1: Temperiergut
- 2: Gehäuse
- 3: Deckel
- 4: Basiskammer
- 5: Temperierkammer
- 6: Bedienelemente
- 7: Anzeigeeinheit
- 8: Boden
- 9: 1. Heizelement
- 10: Heizmodul
- 11: Oberseite
- 12: 1. Stellelement
- 13: 2. Stellelement
- 14: 2. Heizelement
- 15,15': Seiten des Temperiergutes
- 16: Schmalseiten
- 17: Unterseite
- 18: Paddelabschnitte
- 19,19',19": Streben/Randstreben
- 20: Verbindungsstrebe
- 21: T-Stück
- 22: Aufnahme
- 23: Hohlzylinder
- 24: Seitenwand
- 25: Rückwand
- 26: Vorderwand
- 27,27': 1. Hälfte/2. Hälfte des Paddelabschnitts
- 28,28': 1. Hälfte/2. Hälfte des Paddelabschnitts
- 29: Gefrorener Kern
- 30: Flüssigkeit
- 31: Ausgangslage
- 32: Öffnung
- 40,40',40": Breitenabschnitte
- 41: Schmalabschnitte
- 44,44': 1. Hälfte/2. Hälfte eines Breitenabschnitts
- S: Schwenkachse
- a: Abstand
- φ_{MAX}: maximaler positiver Stellwinkel
- -φ_{MAX}: maximaler negativer Stellwinkel
- Q_{T}: Quermittelebene
- Φ: positiver Winkel
- -φ: negativer Winkel
- F_{D},F_{D11},F_{D22}: Druckkraft
- F_{D21},F_{D12}: Druckkraft
- A: Ebene der Ausgangslage
- I: Motorstrom
- L_{T}: Längsmittelebene
- b_{T}: Hälftige Breite
- T/2: Halbperiode
- S_{MAX},-S_{MAX}: maximale Auslenkung
- d1,d2: Abstand
- d2/2: halber Abstand
- g1,g2: Abstand
- X1: Symmetrieachse
- Δt1,Δt2,Δt3: Dauer
- 1/T: Frequenz
- t1-t4: Zeitpunkt
- T_{Soll}: Solltemperatur
- t_{A1}-t_{A4}: Zeitpunkte
- A1,-A1,A1',-A1': Auslenkung/Amplitude
- T_{Z1},T_{Z1}': 1. Zeitintervall
- A2,-A2: Auslenkung/Amplitude
- T_{Z2},T_{Z2}': 2. Zeitintervall
- t_{B1},t_{B2}: Zeitpunkt
- Tₖᵣᵢₜ: kritische Temperatur
- 50: Stellelement, große Ausführung
- 51: Stellelement, kreisförmig
- 52: Stellelement, ellipsenförmig
- 53: Stelleelement, rautenförmig
- 54: Paddelabschnitt, rautenförmig
- 55: Stellelement, asymmetrisch
- 56: Paddelabschnitt, asymmetrisch
- T_{D}: Zeitintervall/Zeitdauer

## Patentansprüche

1. Vorrichtung zum Temperieren und Auftauen von einem Temperiergut (1) mit einem Gehäuse (2), in dem
- das Temperiergut (1) anordbar ist,
- ein Heizmodul (10) zur Einbringung von Wärme an mindestens einer Seite (15, 15') des Temperiergutes (1),
- ein Stellorgan, mittels dessen das Temperiergut (1) in Bewegung bringbar ist, angeordnet sind,
dass das Stellorgan als ein mechanisches Stellelement (12, 13) ausgebildet ist, das sich unter idealerweise direkter Anlage an dem Temperiergut (1) erstreckt und das derart ansteuerbar ist, dass es eine periodische und/oder eine nicht-periodische Bewegung ausführt,
dass das mechanische Stellelement (12, 13) zwischen einem Heizelement (9, 14) des Heizmoduls (10) und dem Temperiergut (1) angeordnet ist und dass das mechanische Stellelement (12, 13) derart ansteuerbar ist, dass eine Schwenkbewegung erfolgt, so dass das Temperiergut (1) partiell in unterschiedlichen Wegstrecken aus seiner Erstreckungsebene ausgelenkt wird.

2. Vorrichtung nach Anspruch 1, **dadurch gekennzeichnet, dass** ein zweites Heizelement (14) des Heizmoduls (10) auf einer gegenüberliegenden Seite (15, 15') des Temperiergutes (1) angeordnet ist.

3. Vorrichtung nach Anspruch 2, **dadurch gekennzeichnet, dass** ein erstes mechanisches Stellelement (12) zwischen einem ersten Heizelement (9) des Heizmoduls (10) und dem Temperiergut (1) und ein zweites mechanisches Stellelement (13) zwischen dem zweiten Heizelement (14) des Heizmoduls (10) und dem Temperiergut (1) angeordnet sind.

4. Vorrichtung nach einem der Ansprüche 1 bis 3, **dadurch gekennzeichnet, dass** das Stellelement (12, 13) flächig, vorzugsweise eben, ausgebildet ist.

5. Vorrichtung nach einem der Ansprüche 1 bis 4, **dadurch gekennzeichnet, dass** das Stellelement (12, 13) als ein Strebenelement ausgebildet ist, wobei Streben (19, 19') eine Öffnung (32) umschließen.

6. Vorrichtung nach einem der Ansprüche 1 bis 5, **dadurch gekennzeichnet, dass** das Stellelement (12, 13) über einen gekoppelten Aktor derart ansteuerbar ist, dass das Stellelement (12, 13) zwischen einem maximalen und minimalen Stellwinkel (φ_{MAX}, - φ_{MAX}) hin und her verschwenkt wird.

7. Vorrichtung nach einem der Ansprüche 1 bis 6, **dadurch gekennzeichnet, dass** das Stellelement (12, 13) derart ansteuerbar ist, dass es mit einer Frequenz von 0,1 bis 25 Hz fortlaufend verschwenkt wird.

8. Vorrichtung nach einem der Ansprüche 1 bis 7, **dadurch gekennzeichnet, dass** das Stellelement (12, 13) eine Linearbewegung und/oder Schwenkbewegung mit einer Amplitude im Bereich von +/- 2 mm bis +/- 100 mm, beispielsweise +/- 10 mm bis +/- 30 mm, vorzugsweise +/- 25 mm, ausführt.

9. Vorrichtung nach einem der Ansprüche 1 bis 8, **dadurch gekennzeichnet, dass** das Stellelement (12, 13) einen parallel zu der Schwenkachse (S) verlaufenden Rand (19') aufweist, der ausgehend von der Quermittelebene (Q_{T}) des Temperiergutes (1) in einem Abstand (a) von 0,2 bis 0,7 einer hälftigen Breite (b_{T}) des Temperiergutes (1) entspricht.

10. Vorrichtung nach einem der Ansprüche 1 bis 9, **dadurch gekennzeichnet, dass** an gegenüberliegenden Seiten (15, 15') des Temperiergutes (1) jeweils ein Stellelement (12, 13) anliegt und dass die Stellelemente (12, 13) derart ansteuerbar sind, dass sie synchron oder asynchron um parallel versetzt zueinander angeordnete Schwenkachsen (S) in eine vorzugsweise gleiche Drehrichtung verschwenkt werden.

11. Vorrichtung nach einem der Ansprüche 1 bis 10, **dadurch gekennzeichnet, dass** die Streben (19, 19', 19") des Stellelementes (12, 13) idealerweise aus einem Drahtmaterial bestehen.

12. Vorrichtung nach einem der Ansprüche 1 bis 11, **dadurch gekennzeichnet, dass** das Stellelement (12, 13) mehrere entlang der Schwenkachse (S) versetzt angeordnete Paddelabschnitte (18) mit O-förmigen Streben (19, 19', 19") aufweist.

13. Vorrichtung nach einem der Ansprüche 1 bis 12, **dadurch gekennzeichnet, dass** eine Anlagefläche des Stellelementes (12, 13) an dem Temperiergut (1) und/oder dem Heizelement (9,14) kleiner ist als 10 % einer dem Stellelement (12, 13) zugekehrten Seite (15, 15') des Temperiergutes (1) und/oder einer dem Stellelement (12, 13) zugekehrten Oberseite (11) oder Unterseite (17) des Heizelementes (9, 14).

14. Vorrichtung nach einem der Ansprüche 1 bis 13, **dadurch gekennzeichnet, dass** in dem Stellelement (12, 13) das Heizelement (9, 14) integriert ist, wobei das Heizelement (9, 14) als ein Plattenwärmer mit starren Wärmeflächen ausgebildet ist.

15. Vorrichtung nach einem der Ansprüche 1 bis 14, **dadurch gekennzeichnet, dass** das Temperiergut (1) derart zu dem Stellelement (12, 13) angeordnet ist, dass das Temperiergut (1) den Paddelabschnitt (18) des Stellelementes (12, 13) vollständig oder zumindest teilweise überdeckt.

16. Vorrichtung nach einem der Ansprüche 1 bis 15, **dadurch gekennzeichnet, dass** das Stellelement (12, 13) einen Breitenabschnitt (40, 40', 40") mit einem Paar von in einem großen Abstand (d2) zueinander und parallel zu der Schwenkachse (S) verlaufenden Randstreben (19') aufweist.

17. Vorrichtung nach einem der Ansprüche 1 bis 16, **dadurch gekennzeichnet, dass** die Auslenkung und/oder die Frequenz und/oder die Beschleunigung der Schwenk- oder Linearbewegung des Stellelementes (12, 13) von Betriebs- oder Prozessparametern wie z.B. der aktuellen Temperatur des Temperiergutes (1) in Verbindung mit einer vorgegebenen Schwelltemperatur, und/oder einem vorgegebenen Zeitpunkt des Temperiervorganges und/oder vom Zustand des Temperiergutes (1) und/oder von einer Viskosität des Temperiergutes (1) abhängig ist.

## Claims

1. An apparatus for controlling the temperature of and thawing a temperature-controlled product (1), comprising a housing (2), containing
- the temperature-controlled product (1),
- a heating module (10) for introducing heat to at least one face (15, 15') of the temperature-controlled product (1),
- an actuator, by means of which the temperature-controlled product (1) can be moved,
that the actuator is configured as a mechanical actuation element (12, 13) which extends in ideally direct contact with the temperature-controlled product (1) and which can be actuated so as to perform a periodic and/or non-periodic movement, that the mechanical actuation element (12, 13) is arranged between a heating element (9, 14) of the heating module (10) and the temperature-controlled product (1) and that the actuation element (12, 13) can be actuated such that the actuation element (12, 13) performs a pivoting movement, so that the temperature-controlled product (1) is partly deflected from the plane of extension thereof in different paths.

2. The apparatus according to Claim 1, **characterized in that** a second heating element (14) of the heating module (10) is arranged on an opposite side of the temperature-controlled product (1).

3. The apparatus according to Claim 1 or 2, **characterized in that** a first actuation element (12) is arranged between a first heating element (9) of the heating module (10) and the temperature-controlled product (1) and that a second actuation element (13) is arranged between a second heating element (14) of the heating module (10) and the temperature-controlled product (1).

4. The apparatus according to any of Claims 1 to 3, **characterized in that** the actuation element (12, 13) has an areal, preferably flat, design.

5. The apparatus according to any of Claims 1 to 4, **characterized in that** the actuation element (12, 13) is configured as a rod element, wherein rods (19, 19') surround an opening (32).

6. The apparatus according to any of Claims 1 to 5, **characterized in that** the actuation element (12, 13) can be actuated by a coupled actuator in such a way that the actuation element (12, 13) is pivoted back and forth between a maximum and minimum actuation angle (φ_{MAX}, -φ_{MAX}).

7. The apparatus according to any of Claims 1 to 6, **characterized in that** the actuation element (12, 13) can be actuated so as to be continuously pivoted at a frequency of 0.1 to 25 Hz.

8. The apparatus according to any of Claims 1 to 7, **characterized in that** the actuation element (12, 13) performs a linear movement and/or pivoting movement at an amplitude in the range of +/- 2 mm to +/- 100 mm, for example +/- 10 mm to +/- 30 mm, preferably +/- 25 mm.

9. The apparatus according to any of Claims 1 to 8, **characterized in that** the actuation element (12, 13) has an edge (19') which extends in parallel with the pivot axis (S) and which, at a distance (a) of 0.2 to 0.7, corresponds to a half width (b_{T}) of the temperature-controlled product (1) from the transverse center plane (Q_{T}) of the temperature-controlled product (1).

10. The apparatus according to any of Claims 1 to 9, **characterized in that** an actuation element (12, 13) is in contact with each of the opposite faces (15, 15') of the temperature-controlled product (1), and **in that** the actuation elements (12, 13) can be actuated so as to be synchronously or asynchronously pivoted in preferably the same rotational direction about pivot axes (S) that are arranged in parallel and offset from one another.

11. The apparatus according to any of Claims 1 to 10, **characterized in that** the rods (19, 19', 19") of the actuation element (12, 13) ideally consist of a wire material.

12. The aparatus according to any of Claims 1 to 11, **characterized in that** the actuation element (12, 13) has a plurality of paddle portions (18) that are arranged offset along the pivot axis (S) and have O-shaped rods (19, 19', 19").

13. The apparatus according to any of Claims 1 to 12, **characterized in that** a resting surface area of the actuation element (12, 13) on the temperature-controlled product (1) and/or on the heating element (9, 14) is smaller than 10% of a face (15, 15') of the temperature-controlled product (1) facing the actuation element (12, 13) and/or of an upper face (11) or lower face (17) of the heating element (9, 14) facing the actuation element (12, 13).

14. The apparatus according to any of Claims 1 to 13, **characterized in that** the heating element (9, 14) is integrated in the actuation element (12, 13), wherein the heating element (9, 14) is configured as a plate heater with rigid heating surfaces.

15. The apparatus according to any of Claims 1 to 14, **characterized in that** the temperature-controlled product (1) is arranged in relation to the actuation element (12, 13) such that the temperature-controlled product (1) entirely or at least in part covers the paddle portion (18) of the actuation element (12, 13).

16. The apparatus according to any of Claims 1 to 15, **characterized in that** the actuation element (12, 13) comprises a wide portion (40, 40', 40") with a pair of edge rods (19') that extend at a large distance (d2) from one another and parallel to the pivot axis (S).

17. The apparatus according to any of Claims 1 to 16, **characterized in that** the deflection and/or the frequency and/or the acceleration of the pivoting or linear movement of the actuation element (12, 13) is dependent on operating or process parameters, such as the current temperature of the temperature-controlled product (1), in conjunction with a specified threshold temperature, and/or on a specified point in time of the temperature-control process and/or on the state of the temperature-controlled product (1) and/or or on the viscosity of the temperature-controlled product (1).

## Revendications

1. Dispositif de thermorégulation et de décongélation d'un produit thermorégulé (1) comprenant un boîtier (2), dans lequel
- le produit thermorégulé (1) peut être disposé,
- un module chauffant (10) pour introduire de la chaleur sur au moins un côté (15, 15') du produit thermorégulé (1),
- un organe d'actionnement, au moyen duquel le produit thermorégulé (1) peut être mis en mouvement, sont disposés,
l'organe d'actionnement étant réalisé sous la forme d'un élément d'actionnement (12, 13) mécanique, qui s'étend en s'appuyant idéalement directement contre le produit thermorégulé (1) et qui peut être commandé de manière à exécuter un mouvement périodique et/ou un mouvement non périodique, l'élément d'actionnement (12, 13) mécanique étant disposé entre un élément chauffant (9, 14) du module chauffant (10) et le produit thermorégulé (1) et l'élément d'actionnement (12, 13) mécanique pouvant être commandé de telle manière qu'un mouvement pivotant se produit, de telle sorte que le produit thermorégulé (1) est partiellement dévié de son plan d'extension dans différents tronçons de chemin.

2. Dispositif selon la revendication 1, **caractérisé en ce qu'**un second élément chauffant (14) du module chauffant (10) est disposé sur un côté (15, 15') opposé du produit thermorégulé (1).

3. Dispositif selon la revendication 2, **caractérisé en ce qu'**un premier élément d'actionnement (12) mécanique est disposé entre un premier élément chauffant (9) du module chauffant (10) et le produit thermorégulé (1) et un second élément d'actionnement (13) mécanique est disposé entre le second élément chauffant (14) du module chauffant (10) et le produit thermorégulé (1).

4. Dispositif selon l'une quelconque des revendications 1 à 3, **caractérisé en ce que** l'élément d'actionnement (12, 13) est réalisé à deux dimensions, de préférence plan.

5. Dispositif selon l'une quelconque des revendications 1 à 4, **caractérisé en ce que** l'élément d'actionnement (12, 13) est réalisé sous la forme d'un élément d'entretoise, des entretoises (19, 19') entourant une ouverture (32).

6. Dispositif selon l'une quelconque des revendications 1 à 5, **caractérisé en ce que** l'élément d'actionnement (12, 13) peut être commandé par le biais d'un actionneur couplé de telle manière que l'élément d'actionnement (12, 13) est pivoté dans un mouvement de va-et-vient entre un angle d'actionnement maximal et un angle d'actionnement minimal (φ_{MAX}, -φ_{MAX}).

7. Dispositif selon l'une quelconque des revendications 1 à 6, **caractérisé en ce que** l'élément d'actionnement (12, 13) peut être commandé de manière à être pivoté en continu avec une fréquence de 0,1 à 25 Hz.

8. Dispositif selon l'une quelconque des revendications 1 à 7, **caractérisé en ce que** l'élément d'actionnement (12, 13) exécute un mouvement linéaire et/ou un mouvement pivotant avec une amplitude comprise dans la plage de +/- 2 mm à +/- 100 mm, par exemple +/- 10 mm à +/- 30 mm, de préférence +/- 25 mm.

9. Dispositif selon l'une quelconque des revendications 1 à 8, **caractérisé en ce que** l'élément d'actionnement (12, 13) comprend un bord (19') s'étendant parallèlement à l'axe de pivotement (S), qui, à partir du plan médian transversal (Q_{T}) du produit thermorégulé (1), correspond à une demi-largeur (b_{T}) du produit thermorégulé (1) à une distance (a) de 0,2 à 0,7.

10. Dispositif selon l'une quelconque des revendications 1 à 9, **caractérisé en ce qu'**un élément d'actionnement (12, 13) s'applique contre chacun des côtés (15, 15') opposés du produit thermorégulé (1) et **en ce que** les éléments d'actionnement (12, 13) peuvent être commandés de manière à être pivotés de manière synchrone ou asynchrone dans un sens de rotation de préférence identique autour d'axes de pivotement (S) disposés parallèlement décalés l'un de l'autre.

11. Dispositif selon l'une quelconque des revendications 1 à 10, **caractérisé en ce que** les entretoises (19, 19', 19") de l'élément d'actionnement (12, 13) sont idéalement constituées d'un matériau en fil métallique.

12. Dispositif selon l'une quelconque des revendications 1 à 11, **caractérisé en ce que** l'élément d'actionnement (12, 13) comprend plusieurs parties formant pagaie (18) disposées en décalage le long de l'axe de pivotement (S) avec des entretoises en forme de O (19, 19', 19").

13. Dispositif selon l'une quelconque des revendications 1 à 12, **caractérisé en ce qu'**une surface d'appui de l'élément d'actionnement (12, 13) sur le produit thermorégulé (1) et/ou l'élément chauffant (9, 14) est inférieure à 10 % d'un côté (15, 15') tourné vers l'élément d'actionnement (12, 13) du produit thermorégulé (1) et/ou d'un côté supérieur (11) ou côté inférieur (17) tourné vers l'élément d'actionnement (12, 13) de l'élément chauffant (9, 14).

14. Dispositif selon l'une quelconque des revendications 1 à 13, **caractérisé en ce que** l'élément chauffant (9, 14) est intégré dans l'élément d'actionnement (12, 13), l'élément chauffant (9, 14) étant réalisé sous la forme d'un chauffeur à plaques comprenant des surfaces chauffantes rigides.

15. Dispositif selon l'une quelconque des revendications 1 à 14, **caractérisé en ce que** le produit thermorégulé (1) est disposé de telle manière par rapport à l'élément d'actionnement (12, 13) que le produit thermorégulé (1) recouvre entièrement ou au moins en partie la partie formant pagaie (18) de l'élément d'actionnement (12, 13).

16. Dispositif selon l'une quelconque des revendications 1 à 15, **caractérisé en ce que** l'élément d'actionnement (12, 13) comprend une partie large (40, 40', 40") comprenant une paire d'entretoises de bord (19') s'étendant à une grande distance (d2) l'une de l'autre et parallèlement à l'axe de pivotement (S).

17. Dispositif selon l'une quelconque des revendications 1 à 16, **caractérisé en ce que** la déviation et/ou la fréquence et/ou l'accélération du mouvement pivotant ou linéaire de l'élément d'actionnement (12, 13) sont dépendantes de paramètres de fonctionnement ou de procédé tels que, par exemple, la température actuelle du produit therrnorégulé (1) en liaison avec une température seuil prédéfinie, et/ou un instant prédéfini du processus de thermorégulation et/ou de l'état du produit thermorégulé (1) et/ou d'une viscosité du produit thermorégulé (1).
